# EUROPEAN PATENT SPECIFICATION

(11) **EP 1 551 374 B1**
(45) Date of publication and mention of the grant of the patent: **07.10.2009**
(21) Application number: 03714356.7
(22) Date of filing: 21.03.2003
(51) Int. Cl.: A61K 9/34, A61K 9/36, A61K 9/28

(54) **POLYMER COMPOSITION AND DOSAGE FORMS COMPRISING THE SAME**
POLYMERZUSAMMENSETZUNG SOWIE DIESE ENTHALTENDE DARREICHUNGSFORMEN
COMPOSITION POLYMERE ET FORMES POSOLOGIQUES LA COMPRENANT

(30) Priority: 28.09.2002 WO PCT/US02/31129; 28.09.2002 WO PCT/US02/31117; 28.09.2002 WO PCT/US02/31062; 28.09.2002 WO PCT/US02/31024; 28.09.2002 WO PCT/US02/31163
(43) Date of publication of application: 13.07.2005
(73) Proprietor: McNeil-PPC, Inc., Skillman, New Jersey 08558 (US)
(72) Inventor: CHEN, Jen-Chi, Morrisville, PA 19067 (US); BUNICK, Frank, J., Quakertown, PA 18951 (US); SOWDEN, Harry, S., Glenside, PA 19038 (US)
(74) Representative: Tunstall, Christopher Stephen
(86) International application number: PCT/US2003/008960
(87) International publication number: WO 2004/028514

(56) References cited:
- EP-A- 0 875 245
- WO-A-96/01874
- WO-A-99/09958
- WO-A-99/21551
- P. J. ANTONY; ET AL.: "A New Disintegrant for Pharmaceutical Dosage Forms" DRUG DEVELOPMENT AND INDUSTRIAL PHARMACY, vol. 23, no. 4, 1997, pages 413-415, XP008023130

## Description

### Field of the Invention

This invention relates to a dosage form comprising a shell portion comprising a high molecular weight, water soluble polymer having a cloud point from about 20 to about 90° C, and a gelling polymer.

### Background of the Invention

A variety of cellulosic polymers are known to be useful in the preparation of dosage forms. They are often combined with otherpolymers and used as coatings or shells for dosage forms. For example, WO 01/32150 discloses an edible, hardenable coating composition containing microcrystalline cellulose, carrageenan, and at least one of a strengthening polymer, a plasticizer, a surface active agent or a combination thereof The composition provides a prompt, i.e., immediate, release coating for solid dosage forms and is applied by spray coating.

WO 00/40223 relates to a composition comprising hydroxypropylcellulose and at least one anionic polymer such as carboxymethyl ether salts of cellulose, methacrylic acid polymers and copolymers, carboxyvinyl polymers and copolymers, methacrylic acid polymers and copolymers, carboxyvinyl polymers and copolymers, alginic acid salts, pectinic acid salts, pectic acid salts, carrageenan, agar and carboxylic acid salts of polysaccharides. The ratio of hydroxypropylcellulose to anionic polymer is from 1 :20 to 20:1. The composition is used as an aqueous solution to coat substrates.

U.S. Patent No. 6,358,525 B1 discloses a pharmaceutical composition containing a medicament and a blend of two components. The first component is hydroxypropylcellulose and the second component is at least one other polymer selected from a group that includes carrageenan, agar, and gellan gum. The pharmaceutical composition is formed into a tablet that may be coated with a conventional coating material.

U.S. Patent No. 6,245,356 B1 relates to a sustained release, oral, solid dosage form comprising agglomerated particles of a therapeutically active medicament in amorphous form, a gelling agent, an ionizable gel strength enhancing agent and an inert diluent. The gelling agent preferably comprises xanthan gum and locust bean gum, but may alternatively comprise alginates, carrageenan, pectin, and other compounds. The ionizable gel strength enhancing agent may be a monovalent or multivalent metal cation. The active medicament in amorphous form, gelling agent, ionizable gel strength enhancing agent and an inert diluent are mixed or granulated together and formed into a tablet.

EP 0 875 245 A discloses a composition comprising Klucel HF (hydroxypropylcellulose having a Molecular Weight of 1,150,000) and carrageenan.

WO 99/2155 discloses a monolithic pharmaceutical table comprising a mixture of HPMC K4M (hydroxypropylmethylcellulose having a viscosity between 80 and 120 Pas) and pectin.

WO 99/09958 discloses a tablet for oral administration comprising Methocel A4M (methyl cellulose having a viscosity greater than 1000 centipoise), a disintegrant such as agar and an edible calcium salt.

WO 03/026612 discloses a dosage form substantially free of pores having a diameter of 0.5 to 5.0 microns obtained by moulding comprising at least about 90% of a material flowable between 37°C and 120°C. The flowable material being a film former (such as HPMC) and a thickener (such as carrageenan).

Known compositions comprising water soluble polymers are often difficult to use, for example in coating operations, because their viscosity becomes too high, especially with increasing polymer concentrations, or increasing polymer molecular weight. Spraying and molding processes can be particularly difficult. Accordingly, dilute solutions must be used, resulting in lengthy processing times to build up adequate thickness.

The applicants have now discovered that a composition comprising a combination of a high molecular weight, water soluble polymer having a cloud point from about 20 to about 90°C and a gelling polymer selected from the group consisting of carrageenan, agar, gellan gum, combinations of carrageenan with locust bean gum, and mixtures thereof can be used as the shell of a dosage form. The two polymers can be dispersed in water, optionally with other ingredients, at a temperature above the cloud point of the high molecular weight, water soluble polymer, leaving the high molecular weight, water soluble polymer undissolved and the viscosity of the dispersion manageable. The dispersion flows easily, and sets quickly and strongly at a relatively high temperature due to the presence of the gelling polymer. Cores can advantageously be coated with this composition, preferably by molding, removed quickly from the coating apparatus, and processed further. Dosage forms comprising such a composition advantageously provide modified release of active ingredient contained therein.

### Summary of the Invention

The invention provides a dosage form comprising a core and at least a first shell portion in contact with said core wherein said first shell portion comprises:
(a) 75 to 95 weight percent of a high molecular weight, water soluble polymer having a cloud point from 20 to 90°C that comprises hydroxymethylcellulose having a viscosity from 80 to 120,000 mPas in a 2% aqueous solution; and
(b) 5 to 25 weight percent of a gelling polymer selected from the group consisting of carrageenan, agar, gellan gum, combinations of carrageenan with locust bean gum, and mixtures thereof,
   said first shell portion composition being a solid that is substantially free of pores having a diameter of 0.5 to 5.0 microns and wherein the average thickness of the first shell is from 25 to 500 microns.

The invention also provides the dosage form of claim 1, wherein said dosage form comprises a second shell portion that is compositionally different from the first shell portion.

### Detailed Description of the Invention

As used herein, the term "dosage form" applies to any solid object, semi-solid, or liquid composition designed to contain a specific pre-determined amount (dose) of a certain ingredient, for example an active ingredient as defined below. Suitable dosage forms may be pharmaceutical drug delivery systems, including those for oral administration, buccal administration, rectal administration, topical or mucosal delivery, or subcutaneous implants, or other implanted drug delivery systems; or compositions for delivering minerals, vitamins and other nutraceuticals, oral care agents, flavorants, and the like. Preferably the dosage forms of the present invention are considered to be solid, however they may contain liquid or semi-solid components. In a particularly preferred embodiment, the dosage form is an orally administered system for delivering a pharmaceutical active ingredient to the gastrointestinal tract of a human.

Suitable active ingredients for use in this invention include for example pharmaceuticals, minerals, vitamins and other nutraceuticals, oral care agents, flavorants and mixtures thereof. Suitable pharmaceuticals include analgesics, anti-inflammatory agents, antiarthritics, anesthetics, antihistamines, antitussives, antibiotics, anti-infective agents, antivirals, anticoagulants, antidepressants, antidiabetic agents, antiemetics, antiflatulents, antifungals, antispasmodics, appetite suppressants, bronchodilators, cardiovascular agents, central nervous system agents, central nervous system stimulants, decongestants, oral contraceptives, diuretics, expectorants, gastrointestinal agents, migraine preparations, motion sickness products, mucolytics, muscle relaxants, osteoporosis preparations, polydimethylsiloxanes, respiratory agents, sleep-aids, urinary tract agents and mixtures thereof.

Suitable oral care agents include breath fresheners, tooth whiteners, antimicrobial agents, tooth mineralizers, tooth decay inhibitors, topical anesthetics, mucoprotectants, and the like.

Suitable flavorants include menthol, peppermint, mint flavors, fruit flavors, chocolate, vanilla, bubblegum flavors, coffee flavors, liqueur flavors and combinations and the like.

Examples of suitable gastrointestinal agents include antacids such as calcium carbonate, magnesium hydroxide, magnesium oxide, magnesium carbonate, aluminum hydroxide, sodium bicarbonate, dihydroxyaluminum sodium carbonate; stimulant laxatives, such as bisacodyl, cascara sagrada, danthron, senna, phenolphthalein, aloe, castor oil, ricinoleic acid, and dehydrocholic acid, and mixtures thereof; H2 receptor antagonists, such as famotadine, ranitidine, cimetadine, nizatidine; proton pump inhibitors such as omeprazole or lansoprazole; gastrointestinal cytoprotectives, such as sucraflate and misoprostol; gastrointestinal prokinetics, such as prucalopride, antibiotics for H. pylori, such as clarithromycin, amoxicillin, tetracycline, and metronidazole; antidiarrheals, such as diphenoxylate and loperamide; glycopyrrolate; antiemetics, such as ondansetron, analgesics, such as mesalamine.

In one embodiment of the invention, the active ingredient may be selected from bisacodyl, famotadine, ranitidine, cimetidine, prucalopride, diphenoxylate, loperamide, lactase, mesalamine, bismuth, antacids, and pharmaceutically acceptable salts, esters, isomers, and mixtures thereof.

In another embodiment, the active ingredient is selected from analgesics, antiinflammatories, and antipyretics, e.g. non-steroidal anti-inflammatory drugs (NSAIDs), including propionic acid derivatives, e.g. ibuprofen, naproxen, ketoprofen and the like; acetic acid derivatives, e.g. indomethacin, diclofenac, sulindac, tolmetin, and the like; fenamic acid derivatives, e.g. mefanamic acid, meclofenamic acid, flufenamic acid, and the like; biphenylcarbodylic acid derivatives, e.g. diflunisal, flufenisal, and the like; and oxicams, e.g. piroxicam, sudoxicam, isoxicam, meloxicam, and the like. In one particular embodiment, the active ingredient is selected from propionic acid derivative NSAID, e.g. ibuprofen, naproxen, flurbiprofen, fenbufen, fenoprofen, indoprofen, ketoprofen, fluprofen, pirprofen, carprofen, oxaprozin, pranoprofen, suprofen, and pharmaceutically acceptable salts, derivatives, and combinations thereof. In another particular embodiment of the invention, the active ingredient may be selected from acetaminophen, acetyl salicylic acid, ibuprofen, naproxen, ketoprofen, flurbiprofen, diclofenac, cyclobenzaprine, meloxicam, rofecoxib, celecoxib, and pharmaceutically acceptable salts, esters, isomers, and mixtures thereof.

In another embodiment of the invention, the active ingredient may be selected from pseudoephedrine, phenylpropanolamine, chlorpheniramine, dextromethorphan, diphenhydramine, astemizole, terfenadine, fexofenadine, loratadine, desloratadine, cetirizine, mixtures thereof and pharmaceutically acceptable salts, esters, isomers, and mixtures thereof.

Examples of suitable polydimethylsiloxanes, which include, but are not limited to dimethicone and simethicone, are those disclosed in United States Patent Nos. 4,906,478, 5,275,822, and 6,103,260. As used herein, the term "simethicone" refers to the broader class of polydimethylsiloxanes, including but not limited to simethicone and dimethicone.

The active ingredient or ingredients are present in the dosage form in a therapeutically effective amount, which is an amount that produces the desired therapeutic response upon oral administration and can be readily determined by one skilled in the art. In determining such amounts, the particular active ingredient being administered, the bioavailability characteristics of the active ingredient, the dosing regimen, the age and weight of the patient, and other factors must be considered, as known in the art. Typically, the dosage form comprises at least 1 weight percent, preferably, the dosage form comprises at least 5 weight percent, e.g. at least 25 weight percent of a combination of one or more active ingredients. In one embodiment, a core comprises a total of at least 50 weight percent, e.g. at least 70 weight percent, say at least 80 weight percent (based on the weight of the core) of one or more active ingredients.

The active ingredient or ingredients may be present in the dosage form in any form. For example, the active ingredient may be dispersed at the molecular level, e.g. melted or dissolved, within the dosage form, or may be in the form of particles, which in turn may be coated or uncoated. If the active ingredient is in form of particles, the particles (whether coated or uncoated) typically have an average particle size of 1-2000 microns. In one embodiment, such particles are crystals having an average particle size of 1-300 microns. In another embodiment, the particles are granules or pellets having an average particle size of 50-2000 microns, for example 50-1000 microns, say 100-800 microns.

The composition of the shell comprises a combination of a high molecular weight, water soluble polymer and a gelling polymer. It is a solid and is substantially free of pores having a diameter of 0.5 to 5.0 microns. It may be used as the shell of a dosage form, or a portion of a shell of a dosage form. When combined with one or more active ingredients, it may be made into an edible matrix. Such an edible matrix may be used as a component of a pharmaceutical dosage form, such as the shell of a dosage form, or a portion of the shell of a dosage form, and may optionally be coated with conventional coating materials, as well known in the art.

The high molecular weight, water soluble polymer has a cloud point from 20 to 90° C. Preferably, the high molecular weight, water soluble polymer has a cloud point from 35 to 70° C. The weight average molecular weight of the high molecular weight, water soluble polymer is in the range of 1000 to 2,000,000 g/mole.

The high molecular weight, water soluble polymers comprise hydroxypropylmethyl cellulose.

In one embodiment, the high molecular weight, water soluble polymer comprises hydroxypropyl methyl cellulose having a weight average molecular weight from 140,000 to 1,150,000. The high molecular weight, water soluble polymer comprises hydroxypropyl methylcellulose having a viscosity from 80 to 120,000 mPa s in 2% aqueous solution.

The gelling polymer is selected from the group consisting of carrageenan, agar, gellan gum, combinations of carrageenan with locust bean gum, and mixtures thereof. In one embodiment, the gelling polymer is carrageenan.

The gelling polymer is present in an amount from 5 to 25 percent of the composition. In one embodiment, the weight ratio of high molecular weight, water soluble polymer to gelling polymer in the composition is typically from 75:25 to 95:5. Preferably, the weight ratio of high molecular weight, water soluble polymer in the composition to gelling polymer is from 80:20 to 90:10.

In another embodiment, active ingredient is combined with the ingredients of the composition to form an edible matrix. When active ingredient is present, the level of high molecular weight water soluble polymer in the edible matrix is adjusted downward by the amount of the active ingredient.

The composition or edible matrix, whether used as a shell, or portion of a shell, i.e. "shell portion," may comprise other optional ingredients. In one embodiment, the composition or edible matrix also comprises an inorganic cation. Suitable inorganic cations include pharmaceutically acceptable monovalent, divalent, and trivalent cations. For example, the inorganic cation may be selected from the group consisting of potassium cations, calcium cations, and mixtures thereof.

In another embodiment, the composition or edible matrix also comprises a water-insoluble polymer. Suitable water-insoluble polymers include of ethyl cellulose, cellulose acetate, cellulose acetate butyrate and mixtures thereof.

Such shell may comprise 1 to 75, or 2 to 24, or 5 to 15, weight percent of the total weight of the dosage form. The average thickness of the shell or shell portion is in the range of 50 to 500 microns.

The shell may completely surround the core, or only partially surround the core. Moreover, only one shell portion may comprise the composition or edible matrix of the invention, as further discussed below. For example, in one embodiment a shell comprising a first shell portion and a second shell portion surrounds the core, and the first shell portion comprises the composition or edible matrix of the present invention, while the second shell portion is compositionally different from the first shell portion. In embodiments wherein a first shell portion of a dosage form comprises the composition or edible matrix of the present invention, the weight of said first shell portion may be from 1 to 75, e.g. 1 to 25, or 1 to 10 percent of the weight of the dosage form.

In embodiments in which the composition or edible matrix is employed as a first shell portion, the second shell portion may comprise any suitable materials, and be applied by any suitable method.

The core may be any solid form. The core may prepared by any suitable method, including for example compression or molding. As used herein, "core" refers to a material which is at least partially enveloped or surrounded by another material. Preferably, the core is a self-contained unitary object, such as a tablet or capsule. Typically, the core comprises a solid, for example, the core may be a compressed or molded tablet, hard or soft capsule, suppository, or a confectionery form such as a lozenge, nougat, caramel, fondant, or fat based composition. In certain other embodiments, the core or a portion thereof may be in the form of a semi-solid or a liquid in the finished dosage form. For example the core may comprise a liquid filled capsule, or a semisolid fondant material. In embodiments in which the core comprises a flowable component, such as a plurality of granules or particles, or a liquid, the core preferably additionally comprises an enveloping component, such as a capsule shell, or a coating, for containing the flowable material. In certain particular embodiments in which the core comprises an enveloping component, the shell or shell portions of the present invention are in direct contact with the enveloping component of the core, which separates the shell from the flowable component of the core.

In one embodiment the core is a compressed tablet having a hardness from 0.196 to 2.94 MPa (2 to 30 kp/cm²), e.g. from 0.588 to 2.45 MPa (6 to 25 kp/cm²). "Hardness" is a term used in the art to describe the diametral breaking strength of either the core or the coated solid dosage form as measured by conventional pharmaceutical hardness testing equipment, such as a Schleuniger Hardness Tester. In order to compare values across different size tablets, the breaking strength must be normalized for the area of the break. This normalized value, expressed in MPa (kp/cm²), is sometimes referred in the art as tablet tensile strength. A general discussion of tablet hardness testing is found in Leiberman et al., Pharmaceutical Dosage Forms - Tablets, Volume 2, 2nd ed., Marcel Dekker Inc., 1990, pp. 213 - 217, 327 - 329.

The core may have one of a variety of different shapes. For example, the core may be shaped as a polyhedron, such as a cube, pyramid, prism, or the like; or may have the geometry of a space figure with some non-flat faces, such as a cone, truncated cone, cylinder, sphere, torus, or the like. In certain embodiments, a core has one or more major faces. For example, in embodiments wherein a core is a compressed tablet, the core surface typically has two opposing major faces formed by contact with the upper and lower punch faces in the compression machine. In such embodiments the core surface typically further comprises a "belly-band" located between the two major faces, and formed by contact with the die walls in the compression machine. A core may also comprise a multilayer tablet. Exemplary core shapes that may be employed include tablet shapes formed from compression tooling shapes described by "The Elizabeth Companies Tablet Design Training Manual" (Elizabeth Carbide Die Co., Inc., p. 7 (McKeesport, Pa.)).

The core typically comprises active ingredient and a variety of excipients, depending on the method by which it is made.

In embodiments in which the core is made by compression, suitable excipients include fillers, binders, disintegrants, lubricants, glidants, and the like, as known in the art. A core made by compression may be a single or multi-layer, for example bilayer, tablet.

Suitable fillers for use in making the core by compression include water-soluble compressible carbohydrates such as sugars, which include dextrose, sucrose, maltose, and lactose, sugar-alcohols, which include mannitol, sorbitol, maltitol, xylitol, starch hydrolysates, which include dextrins, and maltodextrins, and the like, water insoluble plastically deforming materials such as microcrystalline cellulose or other cellulosic derivatives, water-insoluble brittle fracture materials such as dicalcium phosphate, calcium phosphate and the like and mixtures thereof.

Suitable binders for making the core by compression include dry binders such as polyvinyl pyrrolidone, hydroxypropylmethylcellulose, and the like; wet binders such as water-soluble polymers, including hydrocolloids such as acacia, alginates, agar, guar gum, locust bean, carrageenan, carboxymethylcellulose, tara, gum arabic, tragacanth, pectin, xanthan, gellan, gelatin, maltodextrin, galactomannan, pusstulan, laminarin, scleroglucan, inulin, whelan, rhamsan, zooglan, methylan, chitin, cyclodextrin, chitosan, polyvinyl pyrrolidone, cellulosics, sucrose, starches, and the like; and derivatives and mixtures thereof.

Suitable disintegrants for making the core by compression, include podium starch glycolate, cross-linked polyvinylpyrrolidone, cross-linked carboxymethylcellulose, starches, microcrystalline cellulose, and the like.

Suitable lubricants for making the core by compression, include long chain fatty acids and their salts, such as magnesium stearate and stearic acid, tale, glycerides and waxes.

Suitable glidants for making the core by compression, include colloidal silicon dioxide, and the like.

In certain embodiment, the core or a portion thereof may optionally comprise release modifying excipients as known in the art. Suitable release-modifying excipients for making the core by compression include swellable erodible hydrophilic materials, insoluble edible materials, pH-dependent polymers, and the like.

Suitable pharmaceutically acceptable adjuvants for making the cores by compression include, preservatives; high intensity sweeteners such as aspartame, acesulfame potassium, sucralose, and saccharin; flavorants; colorants; antioxidants; surfactants; wetting agents; and the like and mixtures thereof.

In embodiments wherein the core is prepared by compression, a dry blending (i.e. direct compression), or wet granulation process may be employed, as known in the art. In a dry blending (direct compression) method, the active ingredient or ingredients, together with the excipients, are blended in a suitable blender, then transferred directly to a compression machine for pressing into tablets. In a wet granulation method, the active ingredient or ingredients, appropriate excipients, and a solution or dispersion of a wet binder (e.g. an aqueous cooked starch paste, or solution of polyvinyl pyrrolidone) are mixed and granulated. Alternatively a dry binder may be included among the excipients, and the mixture may be granulated with water or other suitable solvent. Suitable apparatuses for wet granulation are known in the art, including low shear, e.g. planetary mixers; high shear mixers; and fluid beds, including rotary fluid beds. The resulting granulated material is dried, and optionally dry-blended with further ingredients, e.g. adjuvants and/or excipients such as for example lubricants, colorants, and the like. The final dry blend is then suitable for compression. Methods for direct compression and wet granulation processes are known in the art, and are described in detail in, for example, Lachman, et al., The Theory and Practice of Industrial Pharmacy, Chapter 11 (3rd ed. 1986).

The dry-blended, or wet granulated, powder mixture is typically compacted into tablets using a rotary compression machine as known in the art, such as for example those commercially available from Fette America Inc., Rockaway, NJ, or Manesty Machines LTD, Liverpool, UK. In a rotary compression machine, a metered volume of powder is filled into a die cavity, which rotates as part of a "die table" from the filling position to a compaction position where the powder is compacted between an upper and a lower punch to an ejection position where the resulting tablet is pushed from the die cavity by the lower punch and guided to an ejection chute by a stationary "take-off" bar.

In one optional embodiment, the core may be prepared by the compression methods and apparatus described in copending U.S. patent Application Serial No. 09/966,509, pages 16-27. Specifically, the core is made using a rotary compression module comprising a fill zone, insertion zone, compression zone, ejection zone, and purge zone in a single apparatus having a double row die construction as shown in Figure 6 of U.S. patent application Serial No. 09/966,509. The dies of the compression module are preferably filled using the assistance of a vacuum, with fillers located in or near each die.

The shell may be substantially unitary and continuous, or the shell may comprise multiple portions, e.g. a first shell portion and a second shell portion. In certain embodiments, at least one such shell portion comprises the composition or edible matrix of the invention. In certain embodiments the shell or shell portions are in direct contact with the core. In certain other embodiments, the shell or shell portions are in direct contact with a subcoating that substantially surrounds the core. In certain embodiments, the shell or a shell portion may comprise one ore more openings therein.

In embodiments in which the shell or shell portion is applied to the core by molding, at least a portion of the shell surrounds the core such that the shell inner surface resides substantially conformally upon the core outer surface. As used herein, the term "substantially conformally" shall mean that the inner surface of the shell has peaks and valleys or indentations and protrusions corresponding substantially inversely to the peaks and valleys of the outer surface of the core. In certain such embodiments, the indentations and protrusions typically have a length, width, height or depth in one dimension of greater than 10 microns, say greater than 20 microns, and less than 30,000 microns, preferably less than 2000 microns.

In certain embodiments, the shell comprises a first shell portion and a second shell portion that are compositionally different. In one embodiment, a first shell portion comprises the composition or edible matrix described herein, and a second shell portion is compositionally different from the first shell portion. As used herein, the term "compositionally different" means having feature that are readily distinguishable by qualitative or quantitative chemical analysis, physical testing, or visual observation. For example, the first and second shell portions may contain different ingredients, or different levels of the same ingredients, or the first and second shell portions may have different physical or chemical properties, different functional properties, or be visually distinct. Examples of physical or chemical properties that may be different include hydrophylicity, hydrophobicity, hygroscopicity, elasticity, plasticity, tensile strength, crystallinity, and density. Examples of functional properties which may be different include rate and/or extent of dissolution of the material itself or of an active ingredient therefrom, rate of disintegration of the material, permeability to active ingredients, permeability to water or aqueous media, and the like. Examples of visual distinctions include size, shape, topography, or other geometric feature, color, hue, opacity, and gloss.

In one embodiment, the dosage form of the invention comprises: a) a core containing an active ingredient; b) an optional subcoating that substantially covers the core; and c) a shell comprising first and second shell portions residing on the surface of the subcoating, the first shell portion comprising the composition or edible matrix described herein. As used herein, "substantially covers" shall mean at least about 95 percent of the surface area of the core is covered by the subcoating.

The use of subcoatings is well known in the art and disclosed in, for example, United States Patent Nos. 3,185,626. Any composition suitable for film-coating a tablet may be used as a subcoating. Examples of suitable subcoatings are disclosed in United States Patent Nos. 4,683,256, 4,543,370, 4,643,894, 4,828,841, 4,725,441, 4,802,924, 5,630,871, and 6,274,162. Additional suitable subcoatittg,s include one or more of the following ingredients: cellulose ethers such as hydroxypropylmethylcellulose, hydroxypropylcellulose, and hydroxyethylcellulose; polycarbohydrates such as xanthan gum, starch, and maltodextrin; plasticizers including for example, glycerin, polyethylene glycol, propylene glycol, dibutyl sebecate, triethyl citrate, vegetable oils such as castor oil, surfactants such as polysorbate-80, sodium lauryl sulfate and dioctyl-sodium sulfosuccinate; polycarbohydrates, pigments, and opacifiers.

In one embodiment, the subcoating comprises from 2 percent to 8 percent, e.g. from 4 percent to 6 percent of a water-soluble cellulose ether and from 0.1 percent to 1 percent, castor oil, as disclosed in detail in United States Patent No. 5,658, 589. In another embodiment, the subcoating comprises from 20 percent to 50 percent, e.g., from 25 percent to 40 percent of HPMC; from 45 percent to 75 percent, e.g., from 50 percent to 70 percent of maltodextrin; and from 1 percent to 10 percent, e.g., from 5 percent to 10 percent of PEG 400.

The dried subcoating typically is present in an amount, based upon the dry weight of the core, from 0 percent to 5 percent.

In one embodiment, an aqueous dispersion of the composition comprising the high molecular weight, water soluble polymer and the gelling polymer is used to prepare the shell. In particular, the high molecular weight, water soluble polymer and gelling polymer are dispersed in water at a temperature above the cloud point of the high molecular weight, water soluble polymer- The dispersion is applied to a core, by for example molding, dipping, spraying, or other means. Preferably, the dispersion is applied to the core by molding. Spraying is least preferred. After application of the dispersion to the core, the core is cooled, preferably at a relatively high temperature, i.e., above the cloud point of the high molecular weight, water soluble polymer.

The aqueous dispersion typically comprises 15 to 40 weight percent solids. In one embodiment, the aqueous dispersion comprises 15 to 20 weight percent solids.

In one embodiment, the high molecular weight, water soluble polymer comprises 15 to 19 weight percent of the total weight of the aqueous dispersion.

In another embodiments, the gelling polymer comprises 1 to 5 weight percent of the total weight of the aqueous dispersion.

The shell thickness at various locations may be measured using a microscope, for example, an environmental scanning electron microscope, model XL 30 ESEM LaB6, Philips Electronic Instruments Company, Mahwah, WI. The shell thickness is measured at 6 different locations on a single dosage form. The relative standard deviation (RSD) is calculated as the sample standard deviation, derided by the mean, times 100 as known in the art (i.e. the RSD is the standard deviation expressed as a percentage of the mean)- The RSD in shell thickness provides an indication of the variation in the thickness of the shell on a single dosage form. In certain optional embodiments of the invention, the relative standard deviation in shell thickness is less than 40%, e.g less than 30%, or less than 20%.

The shell itself or an outer coating thereon may optionally contain active ingredient. In one embodiment, such active ingredient will be released immediately from the dosage form upon ingestion, or contacting of the dosage form with a liquid medium. In another embodiment, such active ingredient will be released in a controller, sustained, prolonged, or extended fashion upon ingestion, or contacting of the dosage form with a liquid medium.

In certain embodiments of the invention, the core, the shell, or the edible matrix are prepared by molding. In such embodiments, the core, the shell, or the edible matrix is made from a dispersion as described above optionally comprising active ingredient. The dispersion comprises the high molecular weight water soluble polymer dispersed in a liquid carrier comprising the gelling agent and a liquid plasticizer at a temperature above the cloud point of the high molecular weight polymers and above the gelling temperature of the gelling polymer. Suitable liquid plasticizers include water, glycerin, propylene glycol, triacetin, triethyl citrate, polyethylene glycol, sorbitol, tribuyl citrate, and mixtures thereof.

In one embodiment, molding is performed via thermal setting molding using the method and apparatus described in copending U.S. patent application Serial No. 09/966,450, pages 57-63. In this embodiment, a core, the shell, or the edible matrix is formed by injecting the dispersion into a molding chamber. The dispersion is cooled and solidifies in the molding chamber into a shaped form (i.e., having the shape of the mold).

According to this method, the dispersion may comprise solid particles of the high molecular weight water-soluble polymer suspended in a liquid carrier comprising the gelling polymer and the liquid plasticizer, e.g. water. Here, the gelling polymer is dissolved in the liquid plasticizer.

In another embodiment, molding is performed by thermal cycle molding using the method and apparatus described in copending U.S. patent application Serial No. 09/966,497, pages 27-51. Thermal cycle molding is performed by injecting the dispersion into a heated molding chamber, In this embodiment, the dispersion may comprise the high molecular weight water soluble polymer dispersed in a liquid carrier comprising the gelling polymer and water at a temperature above the cloud point of the high molecular weight polymer and above the gelling temperature of the gelling polymer. The dispersion is cooled and solidifies in the molding chamber into a shaped form (i.e., having the shape of the mold).

In the thermal cycle molding method and apparatus of U.S. patent application Serial No. 09/966,497 a thermal cycle molding module having the general configuration shown in Figure 3 therein is employed. The thermal cycle molding module 200 comprise a rotor 202 around which a plurality of mold units 204 are disposed. The thermal cycle molding module includes a reservoir 206 (see Figure 4) for holding dispersion. In addition, the thermal cycle molding module is provided with a temperature control system for rapidly heating and cooling the mold units. Figures 55 and 56 depict the temperature control system 600.

The mold units may comprise center mold assemblies 212, upper mold assemblies 214, and lower mold assemblies 210, as shown in Figures 26-28, which mate to form mold cavities having a desired shape, for instance of a core or a shell surrounding one or more cores. As rotor 202 rotates, opposing center and upper mold assemblies or opposing center and lower mold assemblies close. Dispersion, which is heated to a flowable state in reservoir 206, is injected into the resulting mold cavities. The temperature of the dispersion is then decreased, hardening the dispersion. The mold assemblies open and eject the finished product.

In one optional embodiment of the invention, the shell is applied to the dosage form using a thermal cycle molding apparatus of the general type shown in Figures 28A-C of copending U.S. Application Serial No. 09/966,497 comprising rotatable center mold assemblies 212, lower mold assemblies 210 and upper mold assemblies 214. Cores are contiguously fed to the mold assemblies. Dispersion for making the shell, which is heated to a flowable state in reservoir 206, is injected into the mold cavities created by the closed mold assemblies holding the cores. The temperature of the shell dispersion is then decreased, hardening it around the cores. The mold assemblies open and eject the finished dosage forms. Shell coating is performed in two steps, each half of the dosage forms being coated separately as shown in the flow diagram of Figure 28B of copending U.S. Application Serial No. 09/966,939 via rotation of the center mold assembly.

In one embodiment, the compression module of copending U.S. patent application Serial No. 09/966,509, pp. 16-27 may be employed to make the core and the shell is applied to the core using a thermal cycle molding module as described above. A transfer device as described in U.S- patent application Serial No. 09/966,414, pp. 51-57, may be used to transfer the cores from the compression module to the thermal cycle molding module. Such a transfer device may have the structure shown as 300 in Figure 3 of copending U.S. Application Serial No. 09/966,939. It comprises a plurality of transfer units 304 attached in cantilever fashion to a belt 312 as shown in Figures 68 and 69 of copending U.S. Application Serial No. 09/966,939. The transfer device rotates and operates in sync with the compression module and the thermal cycle molding module to which it is coupled. Transfer units 304 comprise retainers 330 for holding cores as they travel around the transfer device.

In certain embodiments wherein a liquid carrier for the dispersion is formed from a mixture of gelling polymer and liquid plasticizer, the liquid carrier may be a thermoplastic system. For example when the gelling polymer is melted and mixed with the liquid plasticizer in a certain ratio, the mixture can be in a thermoplastic state depending on temperature and pressure. In certain other embodiments, the liquid carrier is not a thermoplastic system.

In certain optional embodiments the shell, core, or edible matrix of the invention may additionally comprise a water insoluble polymer at a level of up to about 40%, e.g 15% of the weight of the shell, core, or edible matrix. In embodiments wherein a water insoluble polymer is employed, the weight ratio of high molecular weight water soluble polymer to water insoluble polymer may be from about 99:1 to about 50:50. Suitable water insoluble polymers include ethyl cellulose, cellulose acetate, cellulose acetate butyrate, cellulose propionate, and mixtures thereof.

The dispersion for making cores or the shell by molding may optionally comprise adjuvants or excipients, which may comprise up to about 30% by weight of the dispersion. Examples of suitable adjuvants or excipients include detackifiers, humectants, surfactants, anti-foaming agents, colorants, flavorants, sweeteners, opacifiers, and the like.

In embodiments in which the composition or the edible matrix is prepared by molding, the composition or edible matrix typically is substantially free of pores in the diameter range of 0.5 to 5.0 microns, i.e. has a pore volume in the pore diameter range of 0.5 to 5.0 microns of less than about 0.02 cc/g, preferably less than about 0.01 cc/g, more preferably less than about 0.005 cc/g. Typical compressed materials have pore volumes in this diameter range of more than about 0.02 cc/g. Pore volume, pore diameter and density may be determined using a Quantachrome Instruments PoreMaster 60 mercury intrusion porosimeter and associated computer software program known as "Porowin." The procedure is documented in the Quantachrome Instruments PoreMaster Operation Manual. The PoreMaster determines both pore volume and pore diameter of a solid or powder by forced intrusion of a non-wetting liquid (mercury), which involves evacuation of the sample in a sample cell (penetrometea), filling the cell with mercury to surround the sample with mercury, applying pressure to the sample cell by: (i) compressed air (up to 50 psi maximum); and (ii) a hydraulic (oil) pressure generator (up to 60000 psi maximum). Intruded volume is measured by a change in the capacitance as mercury moves from outside the sample into its pores under applied pressure. The corresponding pore size diameter (d) at which the intrusion takes place is calculated directly from the so-called "Washburn Equation": d= -(4γ(cosθ)/P) where γ is the surface tension of liquid mercury, θ is the contact angle between mercury and the sample surface and P is the applied pressure.

### Equipment used for pore volume measurements:

1. Quantachrome Instruments PoreMaster 60.
2. Analytical Balance capable ofweighing to 0.0001g.
3. Desiccator.

### Reagents used for measurements:

1. High purity nitrogen.
2. Triply distilled mercury.
3. High pressure fluid (Dila AX, available from Shell Chemical Co.).
4. Liquid nitrogen (for Hg vapor cold trap).
5. Isopropanol or methanol for cleaning sample cells.
6. Liquid detergent for cell cleaning.

### Procedure:

The samples remain in sealed packages or as received in the dessicator until analysis. The vacuum pump is switched on, the mercury vapor cold trap is filled with liquid nitrogen, the compressed gas supply is regulated at 55 psi., and the instrument is turned on and allowed a warm up time of at least 30 minutes. The empty penetrometer cell is assembled as described in the instrument manual and its weight is recorded. The cell is installed in the low pressure station and "evacuation and fill only" is selected from the analysis menu, and the following settings are employed:
Fine Evacuation time: 1 min.
Fine Evacuation rate: 10
Coarse Evacuation time: 5 min.

The cell (filled with mercury) is then removed and weighed. The cell is then emptied into the mercury reservoir, and two tablets from each sample are placed in the cell and the cell is reassembled. The weight of the cell and sample are then recorded. The cell is then installed in the low-pressure station, the low-pressure option is selected from the menu, and the following parameters are set:
Mode: Low pressure
Fine evacuation rate: 10
Fine evacuation until: 200µ Hg
Coarse evacuation time: 10 min.
Fill pressure: Contact +0.1
Maximum pressure: 50
Direction: Intrusion And Extrusion
Repeat: 0
Mercury contact angle: 140
Mercury surface tension: 480

Data acquisition is then begun. The pressure vs. cumulative volume-intruded plot is displayed on the screen. After low-pressure analysis is complete, the cell is removed from the low-pressure station and reweighed. The space above the mercury is filled with hydraulic oil, and the cell is assembled and installed in the high-pressure cavity. The following settings are used:
Mode: Fixed rate
Motor speed: 5
Start pressure; 20
End pressure: 60,000
Direction: Intrusion and extrusion
Repeat: 0
Oil fill length: 5
Mercury contact angle: 140
Mercury surface tension: 480

Data acquisition is then begun and graphic plot pressure vs. intruded volume is displayed on the screen. After the high pressure run is complete, the low-and high-pressure data files of the same sample are merged.

Dosage forms according to the invention preferably provide modified release of at least one active ingredient contained therein. As used herein, the term "modified release" means the release of an active ingredient from a dosage form or a portion thereof in other than an immediate release fashion, i.e., other than immediately upon contact of the dosage form or portion thereof with a liquid medium. As known in the art, types of modified release include delayed or controlled. Types of controlled release include prolonged, sustained, extended, retarded, and the like. Modified release profiles that incorporate a delayed release feature include pulsatile, repeat action, and the like. As is also known in the art, suitable mechanisms for achieving modified release of an active ingredient include diffusion, erosion, surface area control via geometry and/or impermeable barriers, and other known mechanism known.

In one preferred embodiment, the composition and edible matrix provide for erosion-based release of ingredients contained therewith. Accordingly, a dosage form in which the composition is employed as the shell may provide delayed release of at least one active ingredient contained in the underlying core. That is, release of the active ingredient from the dosage form is delayed for a pre-determined time after ingestion by the patient. The delay period ("lag time") can be followed either by prompt release of the active ingredient ("delayed burst"), or by sustained (prolonged, extended, or retarded) release of the active ingredients ("delayed then sustained").

The following example further illustrates the invention.

### Example

### A. Preparation of dispersion for making a shell:

Dosage forms of this invention are prepared as follows. A dispersion is Prepared containing 80 parts of hydroxypropyl methylcellulose (HPMC) having a viscosity of about 4000 mPa s in 2% aqueous solution [commercially available from Dow Chemical as METHOCEL K4M]; and 20 parts of Kappa Carrageenan in 471 parts of purified water. The solution has a solids concentration of 17.5%. First, carrageenan is dispersed in room temperature water with an electric mixer equipped with a propellor style blade to form a liquid carrier. Next, the carrageenan/water dispersion is heated to about 80° C with continued mixing. Next, the HPMC is dispersed in the liquid carrier with the propellor mixer, and mixing continued to maintain the HPMC in a suspended state.

### B. Applying the shell to cores:

The dispersion from part A is applied to cores (i.e. MOTRIN 100 mg caplets, available from McNeil-PPC Inc.) using a thermal cycle molding apparatus as described above, to obtain dosage forms having shells residing upon the cores. First, the cores are transferred into a molding chamber as described above. Next, the molding chamber is heated to about 75° C. Next, the dispersion from part A is injected into the heated molding chamber. Next, the thermal cycle molding apparatus is cycled to "cold," and the chamber temperature drops to about 25° C. As the dispersion is cooled, the HPMC dissolves and solidifies in the molding chamber to form a shell around the caplet core. The shell surface takes on the inverse shape and topography of the mold surface. The shell has excellent strength, and is easily removed from the mold.

## Claims

1. A dosage form comprising a core and at least a first shell portion in contact with said core wherein said first shell portion comprises:
(a) 75 to 95 weight percent of a high molecular weight, water soluble polymer having a cloud point from 20 to 90°C that comprises hydroxymethylcellulose having a viscosity from 80 to 120,000 mPas in a 2% aqueous solution; and
(b) 5 to 25 weight percent of a gelling polymer selected from the group consisting of carrageenan, agar, gellan gum, combinations of carrageenan with locust bean gum, and mixtures thereof,
said first shell portion composition being a solid that is substantially free of pores having a diameter of 0.5 to 5.0 microns and wherein the average thickness of the first shell is from 25 to 500 microns.

2. The dosage form of claim 1, wherein said dosage form comprises a second shell portion that is compositionally different from the first shell portion.

3. The dosage form of claims 1 or 2, wherein the shell comprises 1 to 75 percent of the total weight of the dosage form.

4. The dosage form of claim 2, wherein the average thickness of the shell is 25 to 500 microns.

5. The dosage form of claim 1, wherein the cloud point of the high molecular weight, water soluble polymer is 35°C to 70°C.

6. The dosage form of claim 1, wherein the gelling polymer is a high temperature setting anionic polymer.

7. The dosage form of claim 1, wherein the first shell portion further comprises an inorganic cation.

8. The dosage of claim 7, wherein the inorganic cation is selected from the group consisting of potassium cations, calcium cations, and mixtures thereof.

9. The dosage form of claim 1 wherein the first shell portion further comprises a water-insoluble polymer.

10. The dosage form of claim 9, wherein the water-insoluble polymer is selected from the group consisting of ethyl cellulose, cellulose acetate, cellulose acetate butyrate, and mixtures thereof.

11. A process for preparing a dosage form, which comprises coating a core with an aqueous dispersion of a high molecular weight, water soluble polymer having a cloud point from 20 to 90°C that comprises hydroxypropylmethylcellulose having a viscosity from 80 to 120,000 mPas in a 2% aqueous solution in a gelling polymer, selected from the group consisting of carrageenan, agar, gellan gum, combinations of carrageenan and locust bean gum, and mixtures thereof, at a temperature above the cloud point of the high molecular weight, water soluble polymer, and then cooling the coated core;
wherein the weight average molecular weight of the high molecular weight, water soluble polymer is in the range of 1000 to 2,000,000 g/mol.

12. The process of claim 11, wherein the aqueous dispersion comprises 15 to 40 weight percent solids.

13. The process of claim 11, wherein the high molecular weight water soluble polymer comprises 15 to 19 weight percent of the total weight of the aqueous dispersion.

14. The process of claim 11, wherein the gelling polymer comprises 1 to 5 weight percent of the total weight of the aqueous dispersion.

15. The process of claim 11, wherein the aqueous dispersion further comprises ah inorganic cation.

16. The process of claim 15, wherein the inorganic cation is selected from the group consisting of potassium cation, calcium cation, and mixtures thereof.

17. The process of claim 11, wherein the aqueous dispersion further comprises a water-insoluble polymer.

18. The process of claim 17, wherein the water-insoluble polymer is selected from the group consisting of ethyl cellulose, cellulose acetate, cellulose acetate butyrate, and mixtures thereof.

19. The process of claim 11, wherein core is coated with the aqueous dispersion by molding.

## Patentansprüche

1. Darreichungsform, die einen Kern und wenigstens einen ersten Mantelteil in Kontakt mit dem Kern umfasst, wobei der erste Mantelteil:
(a) 75 bis 95 Gewichtsprozent eines wasserlöslichen Polymers mit hohem Molekulargewicht mit einem Trübungspunkt von 20 bis 90°C, das Hydroxymethylcellulose mit einer Viskosität von 80 bis 120.000 mPas in einer 2%-igen wässrigen Lösung umfasst; und
(b) 5 bis 25 Gewichtsprozent eines gelierenden Polymers, das ausgewählt ist aus der Gruppe, die aus Carrageenan, Agar, Gellangummi, Kombinationen von Carrageenan mit Johannisbrotgummi und Mischungen davon besteht,
umfasst, wobei die Zusammensetzung des ersten Mantelteils ein Feststoff ist, der im Wesentlichen frei von Poren mit einem Durchmesser von 0,5 bis 5,0 Mikrons ist, und wobei die durchschnittliche Dicke des ersten Mantels von 25 bis 500 Mikrons beträgt.

2. Darreichungsform nach Anspruch 1, wobei die Darreichungsform einen zweiten Mantelteil umfasst, der im Hinblick auf die Zusammensetzung vom ersten Mantelteil verschieden ist.

3. Darreichungsform nach Ansprüchen 1 oder 2, wobei der Mantel 1 bis 75 Prozent des Gesamtgewichts der Darreichungsform umfasst.

4. Darreichungsform nach Anspruch 2, wobei die durchschnittliche Dicke des Mantels 25 bis 500 Mikrons beträgt.

5. Darreichungsform nach Anspruch 1, wobei der Trübungspunkt des wasserlöslichen Polymers mit hohem Molekulargewicht 35°C bis 70°C beträgt.

6. Darreichungsform nach Anspruch 1, wobei das gelierende Polymer ein bei hoher Temperatur aushärtendes anionisches Polymer ist.

7. Darreichungsform nach Anspruch 1, wobei der erste Mantelteil weiter ein anorganisches Kation umfasst.

8. Darreichungsform nach Anspruch 7, wobei das anorganische Kation ausgewählt ist aus der Gruppe, die aus Kalium-Kationen, Calcium-Kationen und Mischungen davon besteht.

9. Darreichungsform nach Anspruch 1, wobei der erste Mantelteil weiter ein wasserunlösliches Polymer umfasst.

10. Darreichungsform nach Anspruch 9, wobei das wasserunlösliche Polymer ausgewählt ist aus der Gruppe, die aus Ethylcellulose, Celluloseacetat, Celluloseacetatbutyrat und Mischungen davon besteht.

11. Verfahren zur Herstellung einer Darreichungsform, das umfasst, dass ein Kern mit einer wässrigen Dispersion des wasserlöslichen Polymers mit hohem Molekulargewicht mit einem Trübungspunkt von 20 bis 90°C, das Hydroxypropylmethylcellulose mit einer Viskosität von 80 bis 120.000 mPas in einer 2%-igen wässrigen Lösung umfasst, in einem gelierenden Polymer, das ausgewählt ist aus der Gruppe, die aus Carrageenan, Agar, Gellangummi, Kombinationen von Carrageenan und Johannisbrotgummi und Mischungen davon besteht, bei einer Temperatur oberhalb des Trübungspunktes des wasserlöslichen Polymers mit hohem Molekulargewicht beschichtet und dann der beschichtete Kern abgekühlt wird;
wobei das gewichtsgemittelte Molekulargewicht des wasserlöslichen Polymers mit hohem Molekulargewicht im Bereich von 1.000 bis 2.000.000 g/mol liegt.

12. Verfahren nach Anspruch 11, wobei die wässrige Dispersion 15 bis 40 Gewichtsprozent Trockenmasse umfasst.

13. Verfahren nach Anspruch 11, wobei das wasserlösliche Polymer mit hohem Molekulargewicht 15 bis 19 Gewichtsprozent des Gesamtgewichts der wässrigen Dispersion umfasst.

14. Verfahren nach Anspruch 11, wobei das gelierende Polymer 1 bis 5 Gewichtsprozent des Gesamtgewichts der wässrigen Dispersion umfasst.

15. Verfahren nach Anspruch 11, wobei die wässrige Dispersion weiter ein anorganisches Kation umfasst.

16. Verfahren nach Anspruch 15, wobei das anorganische Kation ausgewählt ist aus der Gruppe, die aus Kalium-Kation, Calcium-Kation und Mischungen davon besteht.

17. Verfahren nach Anspruch 11, wobei die wässrige Dispersion weiter ein wasserunlösliches Polymer umfasst.

18. Verfahren nach Anspruch 17, wobei das wasserunlösliche Polymer ausgewählt wird aus der Gruppe, die aus Ethylcellulose, Celluloseacetat, Celluloseacetatbutyrat und Mischungen davon besteht.

19. Verfahren nach Anspruch 11, wobei der Kern mit der wässrigen Dispersion durch Formpressen beschichtet wird.

## Revendications

1. Forme pharmaceutique comprenant un noyau interne et au moins une première partie de coque en contact avec ledit noyau interne, dans laquelle ladite première partie de coque comprend :
(a) 75 à 95 % en poids d'un polymère hydrosoluble, de poids moléculaire élevé ayant un point de rosée de 20 à 90°C qui comprend une hydroxy-méthyl-cellulose ayant une viscosité de 80 à 120 000 mPa dans une solution aqueuse à 2 % ; et
(b) 5 à 25 % en poids d'un polymère gélifiant choisi dans le groupe constitué par les carraghénanes, l'agar, la gomme gellane, les combinaisons de carraghénane avec la farine de graine de caroube, et leurs mélanges,
ladite composition de première partie de coque étant un solide essentiellement dépourvu de pores d'un diamètre de 0,5 à 5,0 microns et dans laquelle l'épaisseur moyenne de la première coque est de 25 à 500 microns.

2. Forme pharmaceutique selon la revendication 1, dans laquelle ladite forme pharmaceutique comprend une seconde partie de coque qui a une composition différente de la première partie de coque.

3. Forme pharmaceutique selon les revendications 1 ou 2, dans laquelle la coque représente de 1 à 75 % du poids total de la forme pharmaceutique.

4. Forme pharmaceutique selon la revendication 2, dans laquelle l'épaisseur moyenne de la coque est de 25 à 500 microns.

5. Forme pharmaceutique selon la revendication 1, dans laquelle le point de rosée du polymère hydrosoluble de poids moléculaire élevé est de 35 à 70°C.

6. Forme pharmaceutique selon la revendication 1, dans laquelle le polymère gélifiant est un polymère anionique thermodurcissable à haute température.

7. Forme pharmaceutique selon la revendication 1, dans laquelle la première partie de coque comprend, en outre, un cation inorganique.

8. Forme pharmaceutique selon la revendication 7, dans laquelle le cation inorganique est choisi dans le groupe constitué par les cations potassium, les cations calcium, et leurs mélanges.

9. Forme pharmaceutique selon la revendication 1, dans laquelle la première partie de coque comprend, en outre, un polymère insoluble dans l'eau.

10. Forme pharmaceutique selon la revendication 9, dans laquelle le polymère insoluble dans l'eau est choisi dans le groupe constitué par l'éthyl-cellulose, l'acétate de cellulose, le butyrate d'acétate de cellulose, et leurs mélanges.

11. Procédé de préparation d'une forme pharmaceutique qui consiste à revêtir un noyau interne avec une dispersion aqueuse d'un polymère hydrosoluble de poids moléculaire élevé ayant un point de rosée de 20 à 90°C qui comprend une hydroxypropyl-méthylcellulose ayant une viscosité de 80 à 120 000 MPa dans une solution aqueuse à 2 % d'un polymère gélifiant, choisi dans le groupe constitué par les carraghénanes, l'agar, la gomme gellane, les combinaisons de carraghénane avec la farine de graine de caroube, et leurs mélanges à une température supérieure au point de rosée du polymère hydrosoluble, de poids moléculaire élevé, puis à refroidir le noyau interne revêtu ; dans lequel le poids moléculaire moyen en poids du polymère hydrosoluble de poids moléculaire élevé est dans la plage de 1000 2 000 000 g/mol.

12. Procédé selon la revendication 11, dans lequel la dispersion aqueuse comprend de 15 à 40 % en poids de fractions solides.

13. Procédé selon la revendication 11, dans lequel le polymère hydrosoluble de poids moléculaire élevé représente de 15 à 19 % en poids du poids total de la dispersion aqueuse.

14. Procédé selon la revendication 11, dans lequel le polymère gélifiant représente de 1 à 5 % en poids du poids total de la dispersion aqueuse.

15. Procédé selon la revendication 11, dans lequel la dispersion aqueuse comprend, en outre, un cation inorganique.

16. Procédé selon la revendication 15, dans lequel le cation inorganique est choisi dans le groupe constitué par le cation potassium, le cation calcium, et leurs mélanges.

17. Procédé selon la revendication 11, dans lequel la dispersion aqueuse comprend, en outre, un polymère insoluble dans l'eau.

18. Procédé selon la revendication 17, dans lequel le polymère insoluble dans l'eau est choisi dans le groupe constitué par l'éthyl-cellulose, l'acétate de cellulose, le butyrate d'acétate de cellulose, et leurs mélanges.

19. Procédé selon la revendication 11, dans lequel le noyau interne est revêtu avec la dispersion aqueuse par moulage.
